# EUROPEAN PATENT APPLICATION

(11) **EP 4 473 921 A1**
(43) Date of publication of application: **11.12.2024**
(21) Application number: 23177330.0
(22) Date of filing: 05.06.2023
(51) Int. Cl.: A61B 17/225, A61B 8/08, A61B 8/00, A61B 17/22, A61B 90/00

(54) **LITHOTRIPTER WITH DETECTION OF KIDNEY STONES**

(71) Applicant: Storz Medical AG, 8274 Tägerwilen (CH)
(72) Inventor: STORZ, Rafael, 8280 Kreuzlingen (CH); BELAU, Markus, 78467 Konstanz (DE); MAZLOUMIAN, Amin, 8044 Zürich (CH); NEUNER, Lukas, 8307 Effretikon (CH); WEISS, Richard, 8408 Winterthur (CH); ROSENTHAL, Matthias, 8157 Dielsdorf (CH)
(74) Representative: Lohr, Jöstingmeier & Partner

(57) **Abstract**

A shock wave and/or ultrasound therapy system has an ultrasound and/or shockwave source with an ultrasonic transducer for ultrasonic imaging or an X-ray imaging system which is configured for providing a video signal, and which is coupled to a video processor. The video processor includes a neural network and is configured for detecting in the video signal at least one kidney stone and/or at least one kidney itself, which are marked in the video signal and displayed on a display. Further, position and/or orientation data of the at least one kidney stone and/or at least one kidney are delivered to a system controller for positioning the ultrasound and/or shockwave source.

## Description

### Field of the invention

The invention relates to an extracorporeal shock wave or ultrasound therapy system, e.g., a lithotripsy system or Lithotripter for non-invasive treatment of stones, like e.g., kidney stones, urinary stones, gallstones, or other calculi within a mammal's body using acoustic pulses, or other applications of an extracorporeal shock wave or ultrasound therapy system.

### Description of the related art

Lithotripters which generate high energy pulses to disintegrate concrements in a human body may have shock wave and/or ultrasound sources within a reflector.

For localizing concrements to be treated, X-ray devices or ultrasound localizing systems using an ultrasonic transducer may be used. Such ultrasonic transducers may be handheld by a surgeon, or they may be fixed in a flexible or adjustable holding device. In EP 0 312 847 A1 a lithotripter with an ultrasonic transducer is disclosed, whereby the transducer is mounted at the center of the shock wave source. To allow for scanning a certain region around the focal point of the shock wave source, the transducer is rotatable and tiltable within the shockwave source.

### Summary of the invention

The problem to be solved by the invention is to provide a lithotripsy system, comprising an ultrasound localizing system which assists a user in detecting and localizing stones, e.g. kidney stones or ureteral stones.

Solutions of the problem are described in the independent claims. The dependent claims relate to further improvements of the invention.

In an embodiment, a shock wave and/or ultrasound device, which may be a lithotripter includes an ultrasound and/or shockwave source which may be within a reflector. Further, an ultrasound imaging sensor is provided, which may either be mounted at the ultrasound and/or shockwave source or may be a separate unit, which may be movable independent from the ultrasound and/or shockwave source.

The ultrasound imaging sensor is connected to a video processor and configured to delivering ultrasound imaging data to the video processor. The video processor is further connected to a display and/or a lithotripter control unit. The video processor includes a neural network which is configured for detecting at least one kidney stone and/or at least one kidney. It evaluates the X-ray or ultrasound image as an input signal, examines it for known structures and then, if the probability of the kidney and/or stone is sufficient, also marks them. The video processor may also be part of a so-called video matrix, which forwards the above-mentioned input signals (videos or images) from the input device to at least one further monitor. Several monitors can also be connected to the output. This video matrix contains enough processing power to examine the input images "on the fly" for the structures and then label and display them as an overlay if the probability is sufficient. This applies to the kidney as well as to a possible stone in the image.

The video processor is further configured for marking the at least one kidney stone and/or at least one kidney in at least one video image which may be forwarded to the display and/or for providing position information of the at least one kidney stone and/or at least one kidney to the lithotripter control unit. The lithotripter control unit may be configured to position the ultrasound and/or shockwave source such, that the at least one kidney stone is in the focus of the ultrasound and/or shockwave source. This may allow for automatic positioning of the shock wave or ultrasound device.

The embodiment helps a surgeon to identify a kidney, a ureteral stone, a kidney stone or other objects in an ultrasound image or video. The embodiment may also identify a bladder.

Detection of a stone may be improved by projecting a shadow of a stone into the image. This allows to increase the probability of detection.

The device may be configured to mark a stone or other concrement by superimposing closed curves on at least one image or a video.

For good ergonomics, the latency time of detecting and marking may be in a range of 1s or below.

Herein, reference is made to the imaging data being video data or a video signal. Such a video signal comprises a sequence of images. The device and method disclosed herein is also applicable for processing of a single image or a plurality of images.

Generally, beyond ultrasound video data, X-ray video data or X-ray images may be processed.

In a further embodiment, the video processor includes a first section including the neural network and being configured for detection of a kidney. The video processor further includes a second section configured to receive information about the kidney from the first section and including an image processor configured for detecting a concrement or kidney stone, which may be in the kidney. Such a detection may be based on a shadow of a stone.

Together with a hexapod drive, the embodiments provide a significant improvement of shock wave and/or ultrasound therapy systems known from prior art. The hexapod drive provides superior flexibility in movement or positioning of the shock wave and/or ultrasound therapy system and/or an ultrasonic transducer for ultrasonic imaging. This allows to position the therapy system and/or the imaging system from different positions and different directions to a concrement or stone to be treated. So, body part, e.g., ribs, which may degrade the ultrasonic view, or the treatment may be avoided. This embodiment works even better, if the hexapod drive is coupled for automatic positioning.

In an embodiment, a shock wave and/or ultrasound device, which may be a lithotripter includes an ultrasound and/or shockwave source which may be within a reflector. Further, an ultrasonic transducer for ultrasonic imaging may be coupled rigidly to the ultrasound and/or shockwave source. It may also be mounted within the reflector. The ultrasonic transducer may be arranged at the center of the ultrasound and/or shockwave source and may further be coaxially to the ultrasound and/or shockwave source. Alternatively, the ultrasonic transducer may be arranged at the center of the reflector and coaxially to the reflector. The ultrasonic transducer may further be oriented towards a focal volume of the shock wave and/or ultrasound device. The ultrasonic transducer may be aligned parallel with the shock wave and/or ultrasound device. The ultrasonic transducer may be configured to generate an image of a part or of a significant portion or all of the focal volume of the shock wave and/or ultrasound device. A focal volume of the shock wave and/or ultrasound device may be indicated in the image of the shock wave and/or ultrasound device. This simplifies targeting or may help to verify automatic targeting. The ultrasonic transducer may be fixedly mounted within the reflector, such that it does not move relative to the reflector.

The ultrasound and/or shockwave source may be suspended on a hexapod drive, such that it can be displaced in at least two or three degrees of freedom and tilted or rotated about one, two or three degrees of freedom. As the hexapod drive allows a free orientation of the source in space, it can basically adjust the ultrasound and/or shockwave source to any required position and orientation. The ultrasonic transducer moves together with the ultrasound and/or shockwave source. Therefore, the ultrasonic transducer can easily be moved for scanning a larger area by moving the ultrasound and/or shockwave source by means of the hexapod drive.

A controller may be provided to control the hexapod drive for specific movements of the ultrasonic transducer, e.g., for scanning a larger area. A larger area scan may include circular movements of the ultrasonic transducer. Further, the controller may be configured for automatically identifying and/or tracking of a concrement.

In an embodiment, 3D control device e.g., a 3D control device may be provided which allows a manual input for movements in 3D space. Such movements may be translation in two or three axes and tilt about one, two or three axes. In an embodiment, there may be a translation in an X'-Y' plane and rotation about a Z'-Axis, perpendicular to the X'-Y' plane in a coordinate system related to the reflector, where the base of the reflector defines the X'-Y' plane and the center axis of the reflector is along the Z'-Axis. The base of the hexapod may be in the X-Y plane of the system with its perpendicular Z-Axis of the shock wave and/or ultrasound therapy system. The movements input by the 3D control device may be forwarded to the hexapod drive such that the ultrasonic transducer performs these movements. This allows for an easy movement of the ultrasonic transducer by a surgeon, for example for scanning a patient and/or for locating a concrement. The ultrasonic transducer may be moved by manual inputs to the 3D control device until the concrement is centered. Then the concrement will automatically be within the focal volume of the ultrasound and/or shockwave source. The controller may be configured to limit the movements of the hexapod drive in spatial relations and /or speed such that too wide or too fast movements are prevented. This may help to protect the patient.

The hexapod drive bearing the ultrasound and/or shockwave source is also known as a hexapod platform. Such a hexapod platform also is called a Stewart platform. Basically, it is a type of parallel manipulator or parallel robot that has six linear actuators, which may be hydraulic or pneumatic jacks or electric linear actuators. Examples of electric linear actors are motors coupled to a belt or a spindle to perform a linear movement. These linear actuators are connected in pairs to three mounting positions at a base, crossing over to three mounting positions at the ultrasound and/or shockwave source. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation and three degrees of rotation. Although the use of six linear actuators is preferred, a lower number of actuators may be used, e.g., like in a delta robot with three actuators.

The hexapod drive allows to adjust the position of an ultrasound and/or shockwave source together with the ultrasonic transducer relative to a patient's body and/or the X-ray system. Positioning of the ultrasound and/or shockwave source may be done automatically or by manual control. An automatic control may allow quick adjustment and it may also allow to store and retrieve preconfigured settings.

Such a hexapod drive is a very robust and mechanical stiff support or suspension of the ultrasound and/or shockwave source. Therefore, it can withstand high forces from the patient body, the weight of the ultrasound and/or shockwave source and dynamic loads which occur when shockwave pulses are generated. Further, a hexapod drive can be moved quickly. Therefore, a stable positioning of the ultrasound and/or shockwave source is achieved providing the additional ability to quickly correct deviations and movements by the patient.

A hexapod drive may allow movement in all 6 degrees of freedom. This allows for a precise adjustment of the position of the focal volume of the ultrasound and/or shockwave source and/or the path of the ultrasound and/or shockwave though the body of the patient.

There may be constraints in the movements, e.g., for safety reasons. In an example, the movement in Z direction may be limited to avoid injuring of the patient.

The ultrasound and/or shockwave source is oriented in a cartesian coordinate system as follows: a y-axis may be a longitudinal axis through the center of a patient table. An x-axis may be orthogonal to the y-axis and in the plane of the table surface. A z-axis is orthogonal to the plane of the table surface and therefore orthogonal to the x-axis and the y-axis and in a direction upward from the table. There may also be a first rotation around the x-axis, a second rotation and a third rotation around the z-axis. A positive rotation may be a clockwise rotation in a direction of a positive axis.

The shock wave or ultrasound therapy system may include a system controller which may control at least the hexapod drive of the ultrasound and/or shockwave source.

The ultrasound and/or shockwave source may be of any type suitable for generating shock waves. It may include a shock wave generator and/or transducer, which may include at least one of a coil, a spark gap, or a Piezo transducer. The shock wave generator/transducer may be partially enclosed by a reflector. Depending on the type of transducer, the reflector may have a parabolic or half-elliptic shape. The ultrasound and/or shockwave source may have a focal volume which is distant from the ultrasound and/or shockwave source and normally around a center axis of the ultrasound and/or shockwave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the ultrasound and/or shockwave source axis of 10 to 15 cm and a diameter between 5 and 15 mm. The focal volume normally is spaced from the shock wave generator and/or transducer.

The patient table may have a basically planar surface defining a longitudinal axis. It is configured for accommodating a patient. The ultrasound and/or shockwave source may be mounted below the patient table. In general, a shockwave source may be mounted in alternative ways, e.g., on a stand or support.

### Description of Drawings

In the following the invention will be described by way of example, without limitation of the general inventive concept, on examples of embodiment with reference to the drawings.
Figure 1 shows an embodiment of a lithotripsy system.
Figure 2 shows a side view.
Figure 3 shows an embodiment in a schematic view.

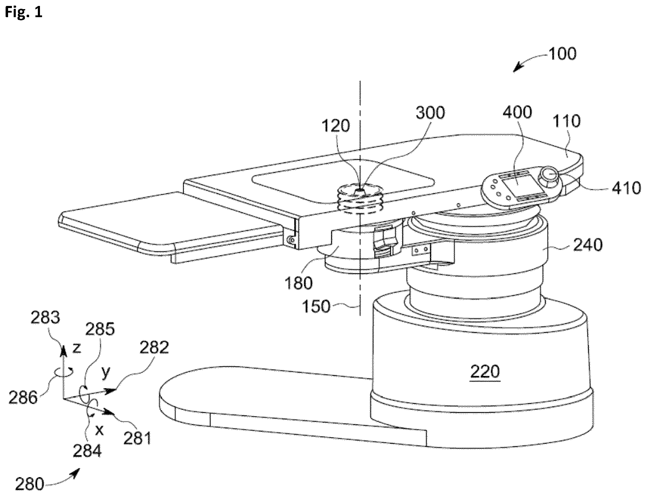

In Figure 1, a first embodiment of a lithotripsy system is shown. The invention works with any ultrasound and/or shockwave treatment system.

An extracorporeal ultrasound and/or shockwave lithotripsy system for non-invasive treatment of stones 100 comprises a patient table 110, an ultrasound and/or shockwave source 120 together with an ultrasonic transducer 300 for ultrasonic imaging. The ultrasound and/or shockwave source 120 may be mounted to a hexapod drive 180. Herein, a hexapod mount and a hexapod drive is given as an exemplary embodiment. Actually, any other mount, e.g., a tiltable and rotatable mount may be used, as the invention is independent of the specific type of mount and would work with all known types of mounts. The hexapod drive 180 may further be held by a stand 220. The hexapod drive 180 allows fine positioning of the ultrasound and/or shockwave source 120 in multiple axes relative to the patient table 110 and therefore relative to the patient (not shown in this figure). The ultrasound and/or shockwave source 120 has a focal volume which may move together with the source. In an embodiment the distance of the focal volume to the source may be modified.

The patient table 110 may be based on a stand 220 which may stand on a floor. This stand may be the same or a different than the stand holding the shockwave source. The table 110 may be held by a positioning device 240 to move the table relative to the ultrasound and/or shockwave source 120 together with the hexapod drive 180. A movement of the patient table may be coarse positioning which may be limited to a displacement in 3 axes.

To describe the relative movement of the table 110, and the ultrasound and/or shockwave source 120, a cartesian coordinate system 280 may be used. There is a y-axis 282, which may be a longitudinal axis 112 through the center of the table. Furthermore, there is an x-axis 281 orthogonal to the y-axis and in the plane of the table surface. A z-axis 283 is orthogonal to the plane of the table surface and therefore orthogonal to the x-axis and the y-axis. There may also be a first rotation 284 around the x-axis, a second rotation 285 and a third rotation 286 around the z-axis. A positive rotation may be a clockwise rotation in a view along a positive axis.

A hexapod drive 180 may allow movement in all these 6 degrees of freedom. This allows for a precise adjustment of the position of the focal volume of the ultrasound and/or shockwave source 120. The patient table may be positioned for a slow and coarse adjustment in 3 degrees of translation for larger distances, but normally no rotation, whereas the hexapod drive provides a comparatively quick adjustment in 3 degrees of translation and 3 degrees of rotation. The movement distances of the table may be larger than the movement distances of the hexapod drive. An ultrasound device may at least be tiltable to perform a second rotation 285 around an axis parallel to the y-axis 282. It may also be tiltable around an axis parallel to the Y axis. It may further be translated in a plane defined by the X and Y axis. This means, it may be moved on the floor which is also in the X-Y plane. Further, it may be rotated along the Z-axis of the shockwave source 120.

For control of the movement of the ultrasound and/or shockwave source 120 relative to the patient table 110, a control panel 400 may be provided, which may include a 3D control device 410, e.g., a control button.

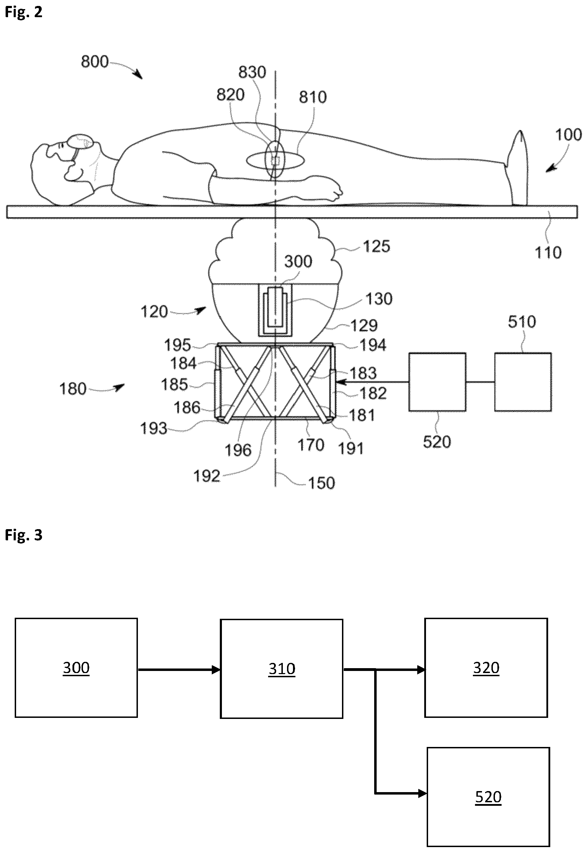

Figure 2 shows a side view. A shock wave or ultrasound device, which may be a lithotripter 100 may include a patient table 110 having a flat surface and an ultrasound and/or shockwave source 120. The ultrasound and/or shockwave source 120 which is shown in a sectional view may be arranged below the patient table 110, such that a patient 800 may be accommodated on top of the patient table. The patient may have a kidney 810 with a kidney stone 820.

A shock wave generator 130 may be held within a reflector 129. In this embodiment, the shock wave generator 130 may include a coil. Centered to the shock wave generator 130 an ultrasonic transducer for ultrasonic imaging 300 may be provided. It may be aligned with the center axis of the ultrasound and/or shockwave source. Such an ultrasonic transducer for ultrasonic imaging may basically be mounted together with the ultrasound and/or shockwave source 120 at the hexapod drive 180, such that both components may be moved together. The ultrasonic transducer for ultrasonic imaging may be configured to generate an image of a part or all of a focal volume 830 of the shock wave and/or ultrasound source 120.

A system controller 510 may be provided to control all system functions. It may also receive input from the control panel 400 and provide user feedback via the control panel. A hexapod control 520 may receive input from the system controller 510 and control the individual actuators of the hexapod drive.

Figure 3 shows a schematic diagram. An ultrasonic transducer for ultrasonic imaging 300 or an X-ray imaging system provides a video signal (imaging data) to a video processor 310. The video processor includes a neural network which is configured for detecting and marking in the video signal at least one kidney stone and/or at least one kidney itself. The video signal with markings is delivered to a display 320. Further position and/or orientation data of the at least one kidney stone and/or at least one kidney may be delivered to a system controller 510 for positioning the ultrasound and/or shockwave source 120. This may allow for automatic positioning of the shock wave or ultrasound device.

### List of reference numerals

- 100: shock wave and/or ultrasound therapy system
- 110: patient table
- 120: ultrasound and/or shockwave source
- 129: reflector
- 130: shock wave generator
- 150: center axis
- 170: base
- 180: hexapod drive
- 181-186: linear actuators
- 191-193: mounting positions at base
- 194-196: mounting positions at ultrasound and/or shockwave source
- 220: stand
- 240: positioning device
- 280: cartesian coordinate system
- 281: x-axis
- 282: y-axis
- 283: z-axis
- 284: tilt around the x-axis
- 285: tilt around the y-axis
- 286: tilt around the z-axis
- 300: ultrasonic transducer for ultrasonic imaging
- 310: video processor
- 320: display
- 400: control panel
- 410: 3D control device
- 510: system controller
- 520: hexapod control
- 800: patient
- 810: kidney
- 820: kidney stone
- 830: focal volume

## Claims

1. A shock wave and/or ultrasound therapy system (100) comprising an ultrasound and/or shockwave source (120) further comprising an ultrasonic transducer for ultrasonic imaging (300) or an X-ray imaging system, **characterized in, that**
the ultrasonic transducer for ultrasonic imaging (300) or an X-ray imaging system, configured for providing a video signal, is coupled to a video processor (310) including a neural network,
wherein the video processor (310) is configured for detecting in the video signal at least one kidney stone and/or at least one kidney itself.

2. The shock wave and/or ultrasound therapy system (100) according to claim 1,
**characterized in, that**
the video processor (310) is configured for marking in the video signal the at least one kidney stone and/or at least one kidney itself, and the video processor (310) video signal is coupled to deliver the video signal with markings to a display (320).

3. The shock wave and/or ultrasound therapy system (100) according to any of the previous claims,
**characterized in, that**
the video processor (310) is configured to deliver position and/or orientation data of the at least one kidney stone and/or at least one kidney to a system controller (510) for positioning the ultrasound and/or shockwave source (120).

4. The shock wave and/or ultrasound therapy system (100) according to any of the previous claims,
**characterized in, that**
the video processor (310) includes:
a first section including the neural network, being configured for detection of a kidney, and
a second section including an image processor configured for detecting a concrement or kidney stone, based on information about the kidney received from the first section.

5. The shock wave and/or ultrasound therapy system (100) according to any of the previous claims,
**characterized in, that**
the ultrasound and/or shockwave source (120) is suspended on a hexapod drive (180).

6. The shock wave and/or ultrasound therapy system (100) according to any of the previous claims,
**characterized in, that**
the ultrasonic transducer for ultrasonic imaging (300) is coupled rigidly to the ultrasound and/or shockwave source (120) and/or the hexapod drive (180) such that movements of the hexapod drive (180) are directly coupled to the ultrasonic transducer.

7. The shock wave and/or ultrasound therapy system (100) according to any of the previous claims,
**characterized in, that**
the ultrasonic transducer for ultrasonic imaging (300) is arranged:
at the center of the ultrasound and/or shockwave source (120) and coaxially to the ultrasound and/or shockwave source (120) or
at the center of the reflector (129) and coaxially to the reflector (129).

8. The shock wave and/or ultrasound therapy system (100) according to any of the previous claims,
**characterized in, that**
the ultrasonic transducer for ultrasonic imaging (300) is aligned parallel with the ultrasound and/or shockwave source (120) and/or
the ultrasonic transducer for ultrasonic imaging (300) is oriented towards a focal volume of the shock wave and/or ultrasound device.

9. The shock wave and/or ultrasound therapy system (100) according to any of the previous claims,
**characterized in, that**
the ultrasonic transducer for ultrasonic imaging (300) is configured to generate an image of a part or all of a focal volume (830) of the shock wave and/or ultrasound source (120) wherein a focal volume of the shock wave and/or ultrasound device may be indicated in the image.

10. The shock wave and/or ultrasound therapy system (100) according to any of the preceding claims,
**characterized in, that**
the hexapod drive (180) includes six linear actuators (181-186) which are attached in pairs to three positions (191-193) at the base (170), crossing over to three mounting positions (194-196) at the ultrasound and/or shockwave source (120).

11. The shock wave and/or ultrasound therapy system (100) according to the preceding claim,
**characterized in, that**
each connection of the linear actuators (181-186) to either the base (170) or the ultrasound and/or shockwave source (120) includes a universal joint or a ball joint.

12. The shock wave and/or ultrasound therapy system (100) according to any of the preceding claims,
**characterized in, that**
the linear actuators (181-186) include at least one of hydraulic or pneumatic jacks or electric linear actuators.

13. The shock wave and/or ultrasound therapy system (100) according to any of the preceding claims,
**characterized in, that**
the hexapod drive (180) is configured to provide movement with at least five degrees of freedom including displacement along three orthogonal axes (281, 282, 283) and at least two degrees of tilt.
